# EUROPEAN PATENT SPECIFICATION

(11) **EP 0 716 651 B1**
(45) Date of publication and mention of the grant of the patent: **03.12.1997**
(21) Application number: 94928010.1
(22) Date of filing: 02.09.1994
(51) Int. Cl.: C07D 213/61

(54) **PRODUCTION OF 2,3,5,6-TETRACHLOROPYRIDINE**
HERSTELLUNG VON 2,3,5,6-TETRACHLORPYRIDIN
PRODUCTION DE LA 2,3,5,6-TETRACHLOROPYRIDINE

(30) Priority: 03.09.1993 IL 10690193
(43) Date of publication of application: 19.06.1996
(73) Proprietor: LUXEMBOURG INDUSTRIES (PAMOL) LTD., 61000 Tel Aviv (IL)
(72) Inventor: SHVO, Youval, 46910 Kfar Shemaryahu (IL)
(74) Representative: Marshall, Monica Anne
(86) International application number: US9410010
(87) International publication number: WO9506639

(56) References cited:
- US-A- 4 327 216
- US-A- 4 360 676

## Description

The present invention relates to a novel, improved process for the production of 2,3,5,6-tetrachloropyridine, also known as Symtet.

2,3,5,6-tetrachloropyridine is useful as an intermediate in the production of various herbicides, fungicides and insecticides, for example the important insecticide O,O-diethyl-O-3,5,6-trichloro-2-pyridyl phosphorothioate.

Various processes are known for the production of 2,3,5,6-tetrachloropyridine. For example, it can be prepared by the liquid phase chlorination of β-picoline (US Patent 4,483,993) or pyridine (US Patent 4,515,953) or by vapor phase chlorination of 2-chloropyridine or 2,6-dichloropyridine (US Patent 3,251,848).

Israel Patent 61581 describes a process whereby a mixture of 2,3,5,6-tetrachloropyridine and 3,5,6-trichloropyridin-2-ol is obtained by reacting trichloroacetyl chloride and acrylonitrile in a solvent at ca 170° C using various metals as catalysts (yields were not reported).

European Patent Application 0 030 215 describes the preparation of 3,3,5-trichloroglutaric acid imide and its subsequent conversion with dehydrating agents such as POCl₃ to 2,3,5,6-tetrachloropyridine.

Unexpectedly, we have discovered that 2,2,4-trichloro-4-cyanobutyrate esters, e.g the methyl or ethyl ester, undergo a one-step conversion to 2,3,5,6-tetrachloropyridine as described in the following equation, where R is an alkyl, aralkyl or aryl group:

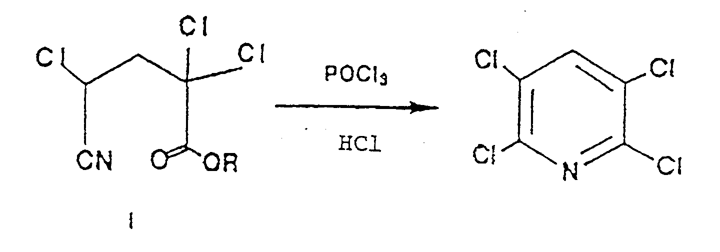

As used herein "aryl" means an aromatic hydrocarbon radical of C₆ to C₁₀ carbon atoms which may optionally be substituted with lower alkyl and/or halo. As used herein "aralkyl" means a C₁-C₆ alkyl group substituted with an aryl radical as defined above.

This novel and remarkably simple transformation results in yields of 60-90% of 2,3,5,6-tetrachloropyridine, depending on the chemical nature of R. The reaction is carried out using POCl₃, a readily available commercial material, at a temperature of at least 100°C, suitably 100 to 160°C, preferably at 120-140° C, in the presence of a catalytic amount of hydrogen chloride. The reaction time is generally 5-10 hours. The molar ratio of POCl₃ to the 2,2,4-trichloro-4-cyanobutyrate ester is generally at least 1 and suitably in the range of 10:1 to 1:1. The preferred range is 2:1 to 3:1. If a high ratio is used, it is recommended to distill out and recycle the excess POCl₃ at the end of the reaction. After the reaction is completed, excess POCl₃ may be distilled off, water is added to the residue and the mixture extracted with a suitable organic solvent, containing now crude 2,3,5,6-tetrachloropyridine. The pure product may be isolated by conventional methods, such as sublimation, crystallization or steam distillation. Product thus obtained exhibited a m.p. 89-90°C; ¹H-NMR (CDCl₃): δ 7.88 ppm (singlet), and was found to be identical in all respects with an authentic sample of 2,3,5,6-tetrachloropyridine.

The hydrogen chloride used preferably comprises dry hydrogen chloride gas. Dry HCl gas in the amount of 0.5-5% by weight of the combined reactant and solvent is recommended. The preferred range is 1.5-3%. The reaction is autocatalytic in the sense that HCl is being produced as the reaction is progressing.

The reaction may also be carried out in the presence of an aprotic and non-reactive solvent, such as aromatic and aliphatic hydrocarbons as well as halogenated hydrocarbons, without seriously affecting the reaction efficiency.

The reaction may be performed at atmospheric pressure or a pressure above atmospheric pressure, e.g. greater than 1 and up to 20 atmospheres.

The starting materials of formula I above, i.e. ethyl 2,2,4-trichloro-4-cyanobutyrate and other esters of 2,2,4-trichloro-4-cyanobutyric acid, can be readily prepared according to methods known in the art which are described in the chemical literature, for example: The Journal of Organic Chemistry, Vol. 29, pp. 2104-5 (1964); and Tetrahedron, Vol. 29, pp. 827-832 (1973); Journal of Organic Chemistry, Vol. 41, pp. 396-398 (1976); and US Patent 5,017,705. Suitably the ester used is derived from a C₁-C₆ alkanol.

The simplicity of the process described herein makes it amenable to large scale production. The high yields obtained with lower alkyl esters render the process attractive from the economical point of view. Although an excess of POCl₃ is generally used, 80% may be recovered and can be recycled. The process is also ecologically sound, as the main by-products in the high yield reactions are phosphoric acid and its esters (the latter can be hydrolyzed to the former).

The invention will now be described in detail by means of the following non-limiting examples.

### Example 1

Ethyl 2,2,4-trichloro-4-cyanobutyrate (12.0 g) and phosphorus oxychloride (40 ml) and dry HCl gas (1.5 g) were placed in a sealed glass reactor that was immersed in a thermo-regulated oil bath at 140°C. After 10 hours at the above temperature, the reactor was cooled to ambient temperature and volatiles were distilled off at 106°C to give 32 ml of POCl₃. Crushed ice was added to the distillation residue, the slurry was stirred for 15 minutes and then extracted with methylene chloride. The methylene chloride was distilled out and the residue was subjected to sublimation at 30-35°C/0.2 mmHg. The collected white crystalline solid, 2,3,5,6-tetrachloropyridine, weighed 9.6 g (90.6% yield), m.p. 88-90°C.

### Example 2

Example 1 was repeated as described above, but the duration of heating at 140°C was reduced to 5 hours. The weight of 2,3,5,6-tetrachloropyridine obtained after sublimation was 7.2 (68% yield).

### Example 3

Phenyl 2,2,4-trichloro-4-cyanobutyrate (12.0g), phosphorus oxychloride (40 ml) and dry HCl gas (1.4 g) were heated at 140°C for 10 hours. The resulting reaction mixture was worked up as described in Example 1. A volume of 33 ml of POCl₃ was recovered. The sublimed product, 2,3,5,6-tetrachloropyridine, weighed 5.34 g (60% yield).

### Example 4

Example 3 was repeated as described above, but the reaction temperature was lowered to 110°C and reaction time was reduced to 5 hours. The methylene chloride solution was evaporated, weighed and analyzed by GLC with an internal standard. The analysis indicated 62% yield of 2,3,5,6-tetrachloropyridine.

### Example 5

Butyl 2,2,4-trichloro-4-cyanobutyrate (12 g), phosphorus oxychloride (40 ml) and dry HCl gas (1.6 g) were heated at 140°C for 10 hours. The resulting reaction mixture was worked up as described in Example 1 and 32 mL of POCl₃ were recovered. The methylene chloride solution obtained after work-up was analyzed by GLC with an internal standard. The analysis indicated a 62% yield of 2,3,5,6-tetrachloropyridine.

### Example 6

p-Tolyl 2,2,4-trichloro-4-cyanobutyrate (12 g), phosphorus oxychloride (40 ml) and dry HCl gas (1.6 g) were heated at 140°C for 10 hours. After cooling to ambient temperature, ice was added and the aqueous slurry was subjected to steam distillation. The white solid was filtered off from the water distillate and dried in vacuum at 60°C to a constant weight. The dried solid, 2,3,5,6-tetrachloropyridine had a m.p. of 87-88°C and weighed 6.03 g (71% yield).

### Example 7

Methyl 4-cyano-2,2,4-trichlorobutyrate (12 g), phosphorus oxychloride (16.5 ml) and xylene (16 ml) were placed in a glass lined reactor which was pressurized with dry HCl to 4 atmospheres, and then heated for 10 hours at 140°C. The pressure increased gradually to 20 atmospheres. The resulting cooled reaction mixture was poured into cold water and subjected to steam distillation. The organic phase of the distillate was separated and assayed (HPLC) for 2,3,5,6-tetrachloropyridine, 9.4 g (83% yield).

### Example 8

Example 7 was repeated, substituting the volume of xylene by an identical volume of cyclohexane. The cooled reaction mixture was poured into cold water, the organic phase separated and the aqueous phase was extracted with 2x15 ml hot cyclohexane. The combined organic extract was washed with sodium carbonate solution, water and assayed (HPLC) for 2,3,5,6-tetrachloropyridine, 9.06 g (80% yield).

### Example 9

Example 8 was repeated, substituting the volume of cyclohexane by an identical volume of 1,2-dichloroethane. After treating the reaction mixture as described in Example 8, there was obtained 2,3,5,6-tetrachloropyridine, 8.8 g (78%).

### Example 10

A mixture of methyl 4-cyano-2,2,4-trichlorobutyrate (12 g), phosphorus oxychloride (20 g) was treated as described in Example 7. After cooling to 70°C, the thick dark reaction mixture was poured into cold water. The resulting slurry was subjected to steam distillation to give white crystalline 2,3,5,6-tetrachloropyridine which, after drying, weighed 8.9 g (79% yield).

## Claims

1. A process for the production of 2,3,5,6-tetrachloropyridine which comprises reacting, at a temperature of at least 100°C, an ester of 2,2,4-trichloro-4-cyanobutyric acid of the general formula I with POCl₃ in the presence of a catalytic amount of hydrogen chloride to produce compound II according to the following reaction: 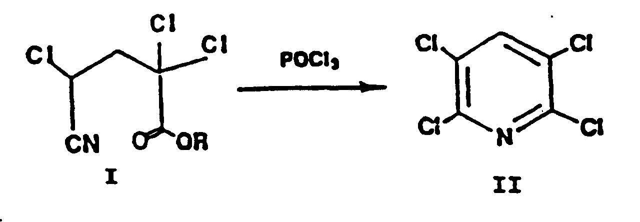 in which R is an alkyl, aryl or aralkyl group.

2. A process according to Claim 1, wherein the hydrogen chloride comprises dry hydrogen chloride gas.

3. A process according to any one of Claims 1 and 2, wherein the ester is derived from a C₁-C₆ alkanol.

4. A process according to any one of Claims 1 and 3, wherein the ester comprises ethyl 2,2,4-trichloro-4-cyanobutyrate.

5. A process according to any one of Claims 1 and 3, wherein the ester comprises methyl 2,2,4-trichloro-4-cyanobutyrate.

6. A process according to any one of Claims 1 to 5, wherein the molar ratio of POCl₃ to the 2,2,4-trichloro-4-cyanobutyrate ester is at least 1.

7. A process according to Claim 6, wherein the molar ratio of POCl₃ to the 2,2,4-trichloro-4-cyanobutyrate ester is in the range of 10:1 to 1:1.

8. A process according to Claim 7, wherein said ratio is in the range of 2:1 to 3:1.

9. A process according to any one of Claims 1 to 8 carried out in the presence of an aprotic inert solvent.

10. A process according to Claim 9, wherein the solvent is selected from aromatic or aliphatic hydrocarbons and halogenated hydrocarbons.

11. A process according to any one of Claims 1 to 10, wherein the reacting is performed at a temperature of 100-160°C.

12. A process according to any one of Claims 1 to 11, wherein the reacting is performed at atmospheric pressure or a pressure above atmospheric pressure.

13. A process according to Claim 12, wherein the pressure is greater than 1 and up to 20 atmospheres.

14. A process according to any one of Claims 1-13, wherein the reacting has a duration of 5 to 10 hours.

## Patentansprüche

1. Verfahren zur Herstellung von 2,3,5,6-Tetrachlorpyridin, umfassend die Umsetzung eines Esters von 2,2,4-Trichlor-4-cyanobuttersäure der allgemeinen Formel I mit POCl₃ bei einer Temperatur von mindestens 100°C in Gegenwart einer katalytischen Menge Chlorwasserstoff zur Erzeugung der Verbindung II, entsprechend der folgenden Gleichung: 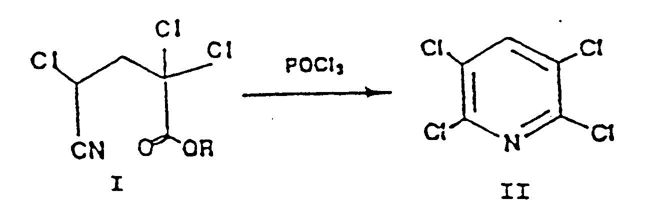 in der R eine Alkyl-, Aryl- oder Aralkylgruppe ist.

2. Verfahren nach Anspruch 1, wobei der Chlorwasserstoff trockenes Chlorwasserstoffgas umfaßt.

3. Verfahren nach einem der Ansprüche 1 und 2, wobei der Ester abgeleitet ist von einem C₁-C₆-Alkanol.

4. Verfahren nach einem der Ansprüche 1 und 3, wobei der Ester Ethyl-2,2,4-trichlor-4-cyanobutyrat umfaßt.

5. Verfahren nach einem der Ansprüche 1 und 3, wobei der Ester Methyl-2,2,4-trichlor-4-cyanobutyrat umfaßt.

6. Verfahren nach einem der Ansprüche 1 bis 5, wobei das Molverhältnis von POCl₃ zu dem 2,2,4-Trichlor-4-cyanobutyratester mindestens 1 beträgt.

7. Verfahren nach Anspruch 6, wobei das Molverhältnis von POCl₃ zu dem 2,2,4-Trichlor-4-cyanobutyratester im Bereich von 10:1 bis 1:1 liegt.

8. Verfahren nach Anspruch 7, wobei das Verhältnis im Bereich von 2:1 bis 3:1 liegt.

9. Verfahren nach einem der Ansprüche 1 bis 8, das in Gegenwart eines aprotischen inerten Lösungsmittels durchgeführt wird.

10. Verfahren nach Anspruch 9, wobei das Lösungsmittel ausgewählt ist aus aromatischen oder aliphatischen Kohlenwasserstoffen und halogenierten Kohlenwasserstoffen.

11. Verfahren nach einem der Ansprüche 1 bis 10, wobei die Umsetzung bei einer Temperatur von 100 bis 160°C durchgeführt wird.

12. Verfahren nach einem der Ansprüche 1 bis 11, wobei die Umsetzung unter Atmosphärendruck oder einem Druck über Atmosphärendruck durchgeführt wird.

13. Verfahren nach Anspruch 12, wobei der Druck größer als 1 und bis zu 20 Atmosphären ist.

14. Verfahren nach einem der Ansprüche 1 bis 13, wobei die Umsetzung 5 bis 10 Stunden lang durchgeführt wird.

## Revendications

1. Procédé pour la production de 2, 3, 5, 6-tétra-chloropyridine qui consiste à faire réagir, à une température d'au moins 100°C, un ester d'acide 2, 2, 4-trichloro-4-cyanobutyrique de formule générale I avec POCl₃ en présence d'une quantité catalytique d'acide chlorhydrique pour produire le composé de formule II selon la réaction suivante : 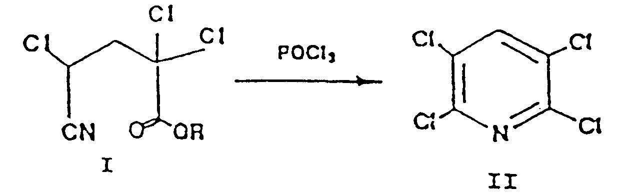 dans laquelle R est un groupe alkyle, aryle ou aralkyle.

2. Procédé selon la revendication 1, dans lequel l'acide chlorhydrique comprend de l'acide chlorhydrique gazeux sec.

3. Procédé selon l'une quelconque des revendications 1 et 2, dans lequel l'ester est dérivé d'un alcanol en C₁-C₆.

4. Procédé selon l'une des revendications 1 et 3, dans lequel l'ester est le 2, 2, 4-trichloro-4-cyanobutyrate d'éthyle.

5. Procédé selon l'une des revendications 1 et 3, dans lequel l'ester est le 2, 2 ,4-trichloro-4-cyanobutyrate de méthyle.

6. Procédé selon l'une quelconque des revendications 1 à 5, dans lequel le rapport molaire du POCl₃ à l'ester d'acide 2, 2, 4-trichloro-4-cyanobutyrique est d'au moins 1.

7. Procédé selon la revendication 6, dans lequel le rapport molaire du POCl₃ à l'ester d'acide 2,2,4-trichloro-4-cyanobutyrique est dans la gamme de 10:1 à 1:1.

8. Procédé selon la revendication 7, dans lequel ledit rapport est dans la gamme de 2:1 à 3:1.

9. Procédé selon l'une quelconque des revendications 1 à 8 caractérisé en ce qu'il est réalisé en présence d'un solvant inerte aprotique.

10. Procédé selon la revendication 9, dans lequel le solvant est choisi parmi les hydrocarbures aromatiques ou aliphatiques et les hydrocarbures halogénés.

11. Procédé selon l'une quelconque des revendications 1 à 10, dans lequel la réaction est effectuée à une température de 100-160°C.

12. Procédé selon l'une quelconque des revendications 1 à 11, dans lequel la réaction est effectuée à pression atmosphérique ou à une pression supérieure à la pression atmosphérique.

13. Procédé selon la revendication 12, dans lequel la pression est supérieure à 1 et jusqu'à 20 atmosphères.

14. Procédé selon l'une quelconque des revendications 1 à 13, dans lequel le temps de réaction est de 5 à 10 heures.
